# EUROPEAN PATENT SPECIFICATION

(11) **EP 2 338 531 B1**
(45) Date of publication and mention of the grant of the patent: **16.01.2013**
(21) Application number: 10460029.1
(22) Date of filing: 28.06.2010
(51) Int. Cl.: A61L 27/12

(54) **Method for fabrication of synthetic bioceramic implant material based on carbonate hydroxyapatites**
Verfahren zur Herstellung von synthetischem biokeramischen Implantatmaterial auf Basis von Kohlenstoff-Hydroxyapatiten
Procédé de fabrication d'un matériau d'implant en biocéramique synthétique sur des dahllites

(30) Priority: 28.12.2009 PL 39004809
(43) Date of publication of application: 29.06.2011
(73) Proprietor: AKADEMIA GORNICZO-HUTNICZA im. Stanislawa Staszica, 30-059 Krakow (PL)
(72) Inventor: Paszkiewicz, Zofia, 30-658 Krakow (PL); Slosarczyk, Anna, 32-091 Michalowice (PL); Zima, Aneta, 30-383 Krakow (PL)
(74) Representative: Magonska, Alina

(56) References cited:
- EP-A1- 0 625 490
- GB-A- 2 349 877
- US-A- 5 952 010
- ZLOSARCZYK A ET AL: "The effect of phosphate source on the sintering of carbonate substituted hydroxyapatite" CERAMICS INTERNATIONAL, ELSEVIER, AMSTERDAM, NL, vol. 36, no. 2, 1 March 2010 (2010-03-01), pages 577-582, XP026808113 ISSN: 0272-8842 [retrieved on 2009-10-29]
- J.C. MERRY ET AL.: "Synthesis and characterisation of carbonate hydroxyapatite" JOURNAL OF MATERIALS SCIENCE: MATERIALS IN MEDICINE, vol. 9, 1 January 1998 (1998-01-01), pages 779-783, XP002603026

## Description

The invention relates to the fabrication of bioceramic implant material based on carbonate hydroxyapatite that can be applied as bone substitute in orthopedics, maxillofacial surgery and preventive dentistry.

Chemical and mineral composition of synthetic hydroxyapatite is close to that of natural bone apatite which is the main inorganic component of bones and teeth. Current studies on hydroxyapatite are focused on its modification with appropriate ions that would result in the material of composition as close as possible to that of natural bone apatite.

Incorporation of C0₃²⁻ groups into the anionic sub-lattice allows to obtain carbonate hydroxyapatite CHAp. CHAp-based bioceramics shows better osteointegrating properties with respect to those exhibited by hydroxyapatite not containing CO₃²⁻ groups.

Magnesium is another possible modifier of hydroxyapatite structure making it closer to the natural one.

Polish patent No 154957 discloses the method for fabrication of ceramic implant material which allows to obtain pure, reactive hydroxyapatite powder from which shaped implant articles are produced..

From Polish patent No 190486 the method for fabrication of highly reactive calcium phosphate powders is known, which involves the one-step precipitation of calcium phosphate deposits by slow addition of H₃PO₄ solution into Ca(OH)₂ suspension, while the amounts of starting regents are such that CaO:P₂O₅ molar ratio is 1,5:1,66, pH of the reaction medium is maintained within 5-11, temperature within 18-90°C. Simultaneously, the reaction suspension is vigorously stirred. Calcium phosphates are formed as gelatinous, amorphous precipitates, in which Ca/P molar ratio is equal to 1,50-1,66. These precipitates are then aged for several dozen hours after which they transform into non-stoichiometric hydroxyapatite with HPO₄⁻² ions in its structure. After filtering, drying and grinding they are calcined at the temperature of 700-900°C, which results in highly reactive powders being a mixture of HAp and TCP or a mono-phase TCP powder.

From the literature (Journal of Material Science : Materials in Medicine 9,1998 , 779-783) the method of preparation of carbonate hydroxyapatite using Ca(NO₃)₂ 4 H₂O, (NH₄)₂HPO₄ and NaHCO₃ (0,2 or 0,4 mole) as starting substances is known. From these powders, shaped articles are produced which are calcined at the temperature of 800-1100°C in CO₂ atmosphere. Dense carbonate hydroxyapatite-based material is thus produced whose relative density is equal to ca. 95%

The paper by Y. Doi et al. (Journal Biomedical Materials Research 39 ,1998 603-610 ) describes a bioresorbable bone substitute fabricated from carbonate hydroxyapatite powders prepared from Ca(NO₃)₂; Na₂HPO₄ and Na₂CO₃.

The powders were uniaxially pressed under the pressure of 600 MPa into shaped articles which were calcined at the temperature of 1200 and 750°C; thus shaped articles of dense CHAp ceramics were prepared. Studies showed close similarity of synthetic carbonate hydroxyapatite to bone apatite.

US 4917702 patent discloses a material for filling bone, teeth and teeth roots defects as well as for fabricating coatings on metallic implants which constitutes apatite powder containing carbonate and alkaline ions in its crystalline lattice.

The publication of A. Slosarczyk et al., Ceramics International 36, 577-582 (2010) discloses a synthesis of carbonate hydroxyapatite by a wet precipitation method using calcium oxide, phosphorous acid and diammonium hydrogen phosphate as reactants. Ammonium hydrogen carbonate is mixed with diammonium hydrogen phosphate and added dropwise during the whole synthesis, while stabilising the pH at 11 or greater using ammonium hydroxide solution. The obtained product is aged, decanted, filtered dried, ground to a size below 0.06 mm and calcined at 900°C.

The present invention has the object of working out a method for fabrication of synthetic hydroxyapatite bioceramics whose properties are similar to those of materials based on natural bone.

The method for fabrication carbonate hydroxyapatite powders in a one-step process involves the synthesis conducted by a drop-wise addition of (NH₄)₂HPO₄ solution, in which a carbonate compound, preferably NH₄HCO₃ and/or NaHCO₃ is dissolved in the amount of 0.05 ― 0.25 M, serving as a source of carbonate ions, C0₃⁻² , as well as of Na⁺ incorporating into hydroxyapatite structure, into an aqueous suspension of Ca(OH)₂ containing a solution of a modifying element salt, preferably magnesium, manganese, sodium, titanium, in the amount of 0 - 0.1 M, while maintaining pH of the reaction medium above 10 using ammonia and the amounts of reagents are such to ensure Ca : P molar ratio equal to 1.55 - 1.95.

Gelatinous, amorphous suspension of non-stoichiometric carbonate hydroxyapatite with incorporated CO₃⁻² , HPO₄⁻² and modifying element ions in its structure is subjected to the aging process, decanted, filtered, then dried and ground which leads to the powder of grain size below 60µm that can be eventually calcined at the temperature of 300 - 500°C.

Powders of carbonate hydroxyapatite modified with various elements obtained are shaped into implant articles using known methods and then are subjected to heat treatment in the temperature range of 600 to 950°C in the presence of carbonate raw materials which introduce CO₂ during sintering.

Implant materials prepared from the powders according to the invention are characterized by ca. 350°C lower sintering temperature, due to which the implant materials produced show lower crystallinity degree, close to that of natural apatites. Lower sintering temperature limits the grain growth which is advantageous for implant mechanical strength. Additionally, the material exhibits high thermal stability (absence of CaO in the calcined material).

Owing to multisubstitutions in cationic and anionic sub-lattices (synergic effect), the prepared material shows similarity to natural bone.

In tests conducted on animals, biomaterial exhibits better osteointegration with respect to HAp not containing CO₃⁻² ions and other modifiers in its structure. Additionally, as a synthetic material, it does not endanger pathogens transmission.

### Example 1

Into Ca(OH)₂ suspension prepared from 0.525 M CaO and 1 liter of distilled water, the solution prepared from 0.3 M (NH₄)₂ HPO₄ , 0.06 M (NH₄)HCO₃ and 0.04M NaHCO₃ is added while maintaining pH of the suspension at ∼ 11 using ammonia. After synthesis, reaction suspension is left unperturbed for several hours during which the aging process and sedimentation of gelatinous precipitate proceed. Then the suspension is decanted and centrifuged, then the densified precipitate is dried in a drier at the temperature of 90°C and ground in a mill to the powder of grain size below 60 µm.

### Example 2

Into Ca(OH)₂ suspension prepared from 0.5 M CaO and 1 liter of 0.006 M solution of magnesium acetate containing 0.03 g manganese acetate, the solution prepared from 0.3 M (NH₄)₂ HPO₄ and 0.1 M (NH₄)HCO₃ is added. Reaction suspension is vigorously stirred at the temperature of 20°C, while its pH is maintained at 10.5-11 using ammonia. After synthesis, suspension of gelatinous precipitate is subjected to the aging process, sedimentation and then the densified suspension after decantation is centrifuged in a centrifuge, while washing the precipitate with distilled water until the filter cake of humidity in the range of 75 - 80 % is formed, which, after drying at the temperature of 90°C, is ground and calcined at the temperature of 500ºC for 1 hour.

### Example 3

Into Ca(OH)₂ suspension prepared from 0.49 M CaO and 1 1 liter of distilled water and 0.008 M magnesium acetate, 0.3 M solution of (NH₄)₂ HPO₄ containing 0.08 M (NH₄)HCO₃ and 0.01 M NaHCO₃ is added drop-wise while maintaining pH of the suspension at the level of 10.5 - 11 using ammonia solution.

After synthesis, suspension of gelatinous precipitate is subjected to the aging process and gravitational sedimentation for 48 hours. Then the suspension is decanted and densified in a centrifuge to a filter cake, after which it is dried and ground to the appropriate grain size fractions, advantageously to the powder of grain size below 60 µm.

From the powder obtained, shaped articles are produced by uniaxial pressing under the pressure of 100 MPa and subjected to thermal treatment. For this purpose, the shaped articles are placed in an alundum crucible with cover into which 30 g of a dolomite powder is added, after which the crucible content is sintered at the rate of 200°C/h, while maintaining at the maximum temperature of 880°C for 1 hour.

### Example 4

From the powder obtained as in Example 3 and after calcination, a slurry is prepared which is then soaked in a polyurethane foam. Shaped articles after drying at 100 °C are placed in an alundum crucible into which 20 g of calcium carbonate was previously added. The sintering process is conducted in an electric oven at the rate of 100°C/h until the temperature of 600°C, and then to 900°C at the rate of 200°C/h, while maintaining at the maximum temperature for 1 hour.

## Claims

1. A method for fabrication of synthetic bioceramic implant material based on carbonate hydroxyapatite **characterized in that** the synthesis is conducted by a drop-wise addition of (NH₄)₂HPO₄ solution in which a carbonate compound is dissolved, preferably NH₄HCO₃ and/or NaHCO₃ in the amount of 0.05 - 0.25 M, serving as a source of CO₃ ⁻² ions as well as Na⁺ incorporating into hydroxyapatite structure, into an aqueous suspension of Ca(OH)₂ containing the solution of a modifying element salt, preferably magnesium, manganese, sodium, titanium in the amount of 0 - 0.1 M, while maintaining pH of the reaction medium above 10 using ammonia and the amounts of the starting compounds are such to ensure Ca : P molar ratio equal to 1.55 - 1.95, the obtained gelatinous suspension of amorphous, non-stoichiometric carbonate hydroxyapatite with CO₃ ⁻², HPO₄⁻² ions and ions of modifying elements incorporated in the structure, is subjected to the aging process, decanted, filtered and then dried and ground which results in the powder of the grain size below 60 µm that is eventually calcined at the temperature of 300 - 500°C.

2. The method of claim 1 **characterized in that** the obtained carbonate hydroxyapatite powders modified with the appropriate elements are shaped into implant articles by known methods, after which they are subjected to thermal treatment in the temperature range of 600 to 950°C in the presence of carbonate raw materials which introduce CO₂ during sintering.

## Patentansprüche

1. Art der Herstellung eines synthetischen, biokeramischen Implantatstoffes auf Grundlage von Carbonat-Hydroxylapatiten, die sich dadurch auszeichnet, dass die Synthese unter tropfenweiser Zugabe zu einer wässrigen Ca(OH)₂-Lösung und gleichzeitiger Zugabe einer Lösung des Salzes des modifizierenden Elements, günstigerweise ein Magnesium-, Mangan-, Natrium- oder Titansalz, in einer Menge von 0 - 0,1 M, einer (NH₄)₂HPO₄ Lösung, in welcher eine Carbonat-Verbindung, am besten NH₄HCO₃ und/oder NaHCO₃ in einer Menge von 0,05 - 0,25 M, aufgelöst wurde und die die Quelle von Carbonat-Ionen CO₃⁻² sowie Na⁺ Ionen darstellt, die sich in die Struktur des Hydroxylapatits einbauen, durchgeführt wird, wobei der pH-Wert der Reaktionsumgebung sich durch die Verwendung von Ammoniak auf einem Niveau von über 10 hält und die Menge der Ausgangsreagenzien derart gewählt wird, dass das Molverhältnis Ca : P 1,55 - 1,95 beträgt. Die entstehende geleeartige, amorphe Suspension des nichtstöchiometrischen Carbonat-Hydroxylapatits, in dessen Struktur die CO₃⁻² und die HPO₄⁻² Ionen sowie die Ionen der modifizierenden Elemente eingebaut sind, wird dem Reifungsprozess unterzogen, dekantiert, gefiltert und anschließend getrocknet und zerkleinert, wodurch ein Pulver mit einer Körnung von unter 60 µm erreicht wird, das anschließend eventuell in einer Temperatur von 300 - 500°C kalziniert wird.

2. Art nach Punkt 1, die sich dadurch auszeichnet, dass aus dem entstehenden Carbonat-Hydroxylapatit-Pulver, das durch entsprechende Elemente modifiziert wurde, durch bekannte Methoden Implantate mit der erforderlichen Form geformt und anschließend einer thermischen Bearbeitung in einem Temperaturbereich zwischen 600°C und 950°C in Anwesenheit von Carbonat-Rohstoffen unterzogen werden, wodurch im Verlaufe der Sinterung CO₂ eingeführt wird.

## Revendications

1. Le procédé de fabrication des matériaux synthétiques biocéramiques pour les implants à base de carbonates hydroxyapatites, **caractérisé en ce que** la synthèse est effectuée en ajoutant dans la suspension de Ca(OH)₂, goutte après goutte et avec l'ajout simultané de la solution de sel d'un élément modifiant, de préférence de magnésium, de sodium, de titane de volume de 0 à 0,1 M, une solution de (NH₄)₂HPO₄ dans laquelle a été dissout un composé de carbonate, de préférence NH₄HCO₃ et/ou NaHCO₃ de volume de 0,05 à 0,25 M qui constitue la source des ions de bicarbonate CO₃⁻² ainsi que de Na⁺ qui s'incorporent à la structure de l'hydroxyapatite, étant entendu que le pH du médium réactionnel est maintenu au niveau supérieur à 10 en utilisant de l'ammoniac et le volume des agents réagissant initiaux est tel que le rapport molaire Ca : P soit de 1,55 à 1,95, la solution gélatineuse obtenue du carbonate hydroxyapatite, amorphe et non-stoechiométrique, dans la structure duquel sont incorporés les ions CO₃⁻², HPO₄⁻² et les ions des éléments modifiants, est soumise au processus de maturation, est décantée, filtrée, ensuite séchée et broyée, ce qui donne comme résultat une poudre de granulométrie inférieure à 60µ qui peut être éventuellement calcinée dans la température de 300 à 500°C.

2. Le procédé selon la revendication 1, **caractérisé en ce que**, avec la poudre obtenue de carbonate hydroxyapatite, modifiée par les éléments appropriés, sont formés des implants de forme requise selon les méthodes connues, qui sont ensuite soumis au traitement thermique dans la température d'ordre de 600 à 950°C en présence de matières premières de carbonate lesquelles introduisent du CO₂ pendant la cuisson.
